# NEW EUROPEAN PATENT SPECIFICATION

(11) **EP 1 931 316 B2**
(45) Date of publication and mention of the opposition decision: **22.02.2017**
(45) Mention of the grant of the patent: 17.02.2010
(21) Application number: 06778240.9
(22) Date of filing: 15.08.2006
(51) Int. Cl.: A61K 9/36, A61K 38/46

(54) **CONTROLLED RELEASE PHARMACEUTICAL COMPOSITIONS FOR ACID LABILE DRUGS**
PHARMAZEUTISCHE ZUSAMMENSETZUNGEN MIT KONTROLLIERTER FREISETZUNG FÜR SÄURELABILE ARZNEIMITTEL
COMPOSITIONS PHARMACEUTIQUES A LIBERATION CONTROLEE DESTINEES A DES MEDICAMENTS ACIDO-LABILES

(30) Priority: 15.08.2005 EP 05107472; 15.08.2005 US 708692 P
(43) Date of publication of application: 18.06.2008
(73) Proprietor: Abbott Laboratories GmbH, 30173 Hannover (DE)
(72) Inventor: SHLIEOUT, George, 31319 Sehnde (DE); KOELLN, Claus-Jürgen, 31535 Neustadt A. Rbge. (DE); SCZESNY, Frithjof, 30173 Hannover (DE); ONKEN, Jens, 30890 Barsinghausen (DE); KOERNER, Andreas, 31832 Springe (DE)
(74) Representative: Hart-Davis, Jason
(86) International application number: PCT/EP2006/065311
(87) International publication number: WO 2007/020259

(56) References cited:
- EP-A- 0 035 780
- US-A- 4 786 505
- US-A1- 2004 101 562
- US-A1- 2004 213 847

## Description

Described herein is a pharmaceutical composition in an oral dosage form and methods for making and using the same. More specifically, described herein are pharmaceutical compositions for acid-labile active pharmaceutical ingredients in an enteric-coated oral dosage form where the dosage form is substantially free of both monomeric phthalic acid ester plasticizers and synthetic oils.

Numerous active pharmaceutical ingredients (API) or drugs are known to be incompatible with the acidic environment present in mammalian, such as human, stomachs. Due to this incompatibility, it can be advantageous to protect these acid-labile compounds until such time as they reach a point in the gastro-intestinal (GI) tract having a pH which is more compatible with the particular API. Controlled or delayed release pharmaceutical compositions for acid-labile drugs, in particular for acid-labile drugs that need to be delivered to the upper intestine of a mammal and where exposure of the acid-labile API to the acidic gastric environment is to be avoided, are often desirable.

One such acid-labile API or acid-labile drug which is advantageously delivered to the human duodenum is pancreatin. Pancreatin is a substance which is derived from mammalian pancreas glands and comprises different digestive enzymes such as lipases, amylases and proteases. Pancreatin has been used to treat pancreatic exocrine insufficiency (PEI) which is often associated with cystic fibrosis, chronic pancreatitis, post-pancreatectomy, post-gastrointestinal bypass surgery (e.g. Billroth II gastroenterostomy) and ductal obstruction from neoplasm (e.g. of the pancreas or common bile duct). Pancreatin microspheres are the treatment of choice for diseases or disorders caused by digestive enzyme deficiency in mammals such as humans. This is due to the fact that high-performance pancreatin microsphere products like Creon™ provide a therapeutically effective load of active enzymes while at the same time providing properly sized microspheres capable of targeting the optimal location in the digestive tract where digestive enzyme activity will be needed, in particular the upper intestine.

Recently, health authorities have initiated a reassessment of the compatibility of certain pharmaceutical excipients which had previously been used in the formulation of pancreatin-containing products. As a result, some health authorities have provided advice concerning the use of specific pharmaceutical excipients (see e.g. US Code of Federal Regulations, 21 CFR §201.302), such as mineral oil and dibutyl phthalate (see e.g. directive 2003/36/EC of the European Parliament and the Council of 26 May 2003 amending for the 25th time Council Directive 76/769/EEC). Consequently, it is now recommended that mineral oil not be provided indiscriminately to either pregnant women or infants. Similarly, health authorities today recommend restricting the use of dibutyl phthalate. Therefore, a need exists to provide patients with formulations of pharmaceutical products which would be responsive to the current advice of health authorities.

Some controlled release pharmaceutical preparations and/or methods for preparing them are disclosed in EP 0063014 or US 5,725,880.

Pharmaceutical preparations which may comprise pancreatin and an enteric coating are disclosed in DE 19907764; EP 0021129 (US 4,280,971); EP 0035780; EP 0583726 (US 5,378,462); US 5,225,202; US 5,750,148; US 6,224,910; US 2002/0146451 and WO 02/40045.

US patent No. 4,786,505 discloses pharmaceutical preparations for oral use.

Published patent application US 2004/0213847 discloses delayed release pharmaceutical compositions containing proton pump inhibitors.

Published patent application US 2002/061302 discloses the use of physiologically acceptable enzyme mixtures for the treatment of diabetes.

Accordingly, one embodiment disclosed herein is an enteric-coated oral dosage form containing an acid-labile API where the dosage form is substantially free of monomeric phthalic acid ester plasticizers and synthetic oils.

It has now been surprisingly found that a controlled release pharmaceutical composition for acid-labile drugs, such as pancreatin, in the upper intestine can be achieved by providing an enteric-coated oral dosage form of an acid-labile drug wherein the enteric-coating comprises at least one plasticizer and at least one film-forming agent as described in more detail below. The new enteric coating as disclosed herein is substantially free of both, monomeric phthalic acid ester plasticizers, such as dibutyl phthalate, and synthetic oils, such as paraffins or mineral oils, while at the same time providing the desired targeted release and storage stability. The enteric coating as disclosed herein further provides beneficial properties which are comparable to the respective properties of pharmaceutical compositions which contains dibutyl phthalate and synthetic oil in the formulation.

It is therefore provided herein an enteric coating comprising
a) at least one film-forming agent **as defined in claim 1**;
b) a plasticizer which is a mixture of cetyl alcohol and triethyl citrate, which are collectively present in an amount of greater than 3% by weight relative to the at least one film-forming agent and wherein the weight to weight ratio of cetyl alcohol to triethyl citrate is from 0.05:1 to 1:1; and
c) optionally at least one anti-sticking agent.

The enteric coating can be applied to oral dosage forms of acid-labile drugs, such as pancreatin, which need to be delivered to the the GI tract at a location having a pH higher than the stomach. By applying the enteric coating as disclosed herein to oral dosage forms of acid-labile drugs, controlled release pharmaceutical compositions (CRPC) of the acid-labile drugs can be produced. **Claim 1 is directed to a CRPC containing pancreatin and having the said enteric coating.**

Film-forming agent(s), plasticizer(s) and anti-sticking agent(s) (when present) as used for preparing the enteric coating are hereinafter commonly referred to as "non-solvent coating constituents".

Cellulose acetate phthalate, hydroxypropyl methylcellulose acetate succinate and/or methacrylic acid-ethyl methacrylate-copolymer are the preferred film-forming agents. Most preferred is hydroxypropyl methylcellulose phthalate, e.g. HP 55 or HPMCP HP-50. Synthetic oils are not to be regarded as preferred film-forming agents.

Synthetic oils and monomeric phthalic acid esters are not to be regarded as suitable plasticizers.

The plasticizer is comprised of cetyl alcohol and triethyl citrate which are collectively present in an amount of greater than 3%, typically in an amount of 4% to 20%, in particular between 6% and 15%, more particularly between 7% and 10%, by weight in relation to the film-forming agent. The weight to weight ratio of cetyl alcohol to triethyl citrate in said mixture of cetyl alcohol and triethyl citrate may be from 0.05:1 to 1:1, for example 0.1:1, 0.2:1, 0.3:1, 0.4:1, 0.5:1, 0.6:1, 0,7:1, 0.8:1 or 0.9:1. In particular, the ratio of cetyl alcohol to triethyl citrate in said mixture of cetyl alcohol and triethyl citrate may be from 0.25:1 to 0.5:1, preferably from 0.3:1 to 0.45:1, more preferably from 0.35:1 to 0.4:1, and even more preferably from 0.38:1 to 0.4:1 (w/w).

The enteric coating optionally comprises an anti-sticking agent. Suitable anti-sticking agents include dimethicone and castor oil. Dimethicone, in particular dimethicone 1000, is the preferred anti-sticking agent. The amount of anti-sticking agent (if present) in the enteric coating is between 1.5% and 3% by weight relative to the film-forming agent. Synthetic oils are not to be regarded as preferred anti-sticking agents. The foregoing list of anti-sticking agents is not meant to be exhaustive but merely illustrative, as a person of ordinary skill in the art would understand that many other anti-sticking agents or combinations of anti-sticking agents could also be used.

In one embodiment the enteric coating comprises between 20% and 30% by weight, more preferably between 22% and 26% by weight, yet more preferably between 22.5 % and 25 % by weight of the total composition of the enteric coated oral dosage form or CRPC.

The phrase "substantially free of synthetic oils" means that the manufacturing processes described herein and used to make the enteric coating or the enteric coated oral dosage forms of acid-labile drugs where applicable do not utilize one or more synthetic oils as an excipient although synthetic oils may be present as pharmaceutically acceptable trace contaminants in the API, binding agent(s), enteric coating constituents, organic solvents and/or excipients which are used to manufacture the enteric coating and/or the enteric coated oral dosage forms of acid-labile drugs described herein.

The phrase "substantially free of monomeric phthalic acid esters" means that the manufacturing processes described herein and used to make the enteric coating or the enteric coated oral dosage forms of acid-labile drugs where applicable do not utilize one or more monomeric phthalic acid esters (e.g. dibutyl phthalate) as an excipient although monomeric phthalic acid esters may be present as pharmaceutically acceptable trace contaminants in the API, binding agent(s), enteric coating constituents, organic solvents and/or excipients which are used to manufacture the enteric coating and/or the enteric coated oral dosage forms of acid-labile drugs described herein.

Pancreatin is a mixture of different physiologically active endogenous ingredients which is derived from mammalian pancreas glands and comprises as main constituents different digestive enzymes like lipases, amylases and proteases. Mammalian pancreatic lipase is typically used as a digestive enzyme supplement or substitute for the treatment of PEI but pancreatic proteases and amylases also contribute to the therapeutic value of pancreatin. Pancreatin for pharmaceutical use is typically of bovine or porcine origin. Porcine pancreatin is preferred.

The oral dosage form containing the acid-labile drug or API may be in the form of, for example, capsules, granules, granulates, micropellets, microspheres, microtablets, pellets, pills, powders and/or tablets. For the purposes of this invention, the prefix "micro" is used to describe an oral dosage form if the diameter of the oral dosage form or all of its dimensions (length, height, width) is equal to or below 5 mm.

Enteric coated granules, granulates, micropellets, microspheres, pellets, pills or powders, if desired may be filled into capsules or sachets or may be compressed to form microtablets or tablets. Equally, uncoated granules, granulates, micropellets, microspheres, pellets, pills or powders may be first compressed to form microtablets or tablets which may then be coated with the enteric coating as provided according to the invention. Microtablets or tablets may likewise be filled into capsules.

Granules are asymmetric agglomerates of powder particles cemented together and having no regular geometric form. The surface of the granule may be spherical, rod-shaped or cylindrical and is usually uneven and ridged. Granules are preferably produced by melt or wet granulation. Granulates are usually defined to be sedimented agglomerates of granules. Tablets are usually made from the powder or the granules.

Pellets and micropellets can be produced either by exploiting the thermoplastic properties of the excipients in a high share mixer (melt pelletisation) or by other methods such as extrusion (e.g. melt extrusion or wet extrusion) and spheronisation. Micropellets and microspheres can in particular be produced by extrusion and spheronisation. Pharmaceutical pellets, micropellets and microspheres are usually of a defined geometrical form and have a generally smooth surface. Specific methods of producing micropellets or microspheres are described herein. Pellets, microspheres and micropellets are the preferred oral dosage forms described herein. Most preferred are microspheres and micropellets wherein pancreatin is the acid-labile drug. Pancreatin micropellets without an enteric coating are sometimes referred to as "pancreatin micropellet cores".

In one preferred embodiment, the oral dosage form is a pancreatin micropellet or pancreatin microsphere which comprises 10% to 95% by weight of pancreatin, 5% to 90% by weight of at least one pharmaceutically acceptable binding agent and 0% to 10% by weight of at least one pharmaceutically acceptable excipient. More specifically, pancreatin micropellets can be produced by the process described below which comprise 70% to 90% by weight of pancreatin, 10% to 30% by weight of at least one pharmaceutically acceptable binding agent and 0% to 5% by weight of at least one pharmaceutically acceptable excipient. In one embodiment, pancreatin micropellets can be produced which comprise 70% to 90% by weight pancreatin, and 10% to 30% by weight of at least one pharmaceutically acceptable binding agent, the constituents adding to 100 % by weight in each case. In one embodiment the pancreatin micropellet or pancreatin microsphere is approximately spherical and has a diameter between 0.5 mm and 2.0 mm (range limits included).

Examples of pharmaceutically acceptable binding agents include polyethylene glycol 1500, polyethylene glycol 2000, polyethylene glycol 3000, polyethylene glycol 4000, polyethylene glycol 6000, polyethylene glycol 8000, polyethylene glycol 10000, hydroxypropyl methylcellulose, polyoxyethylen, copolymers of polyoxyethylen-polyoxypropylen and mixtures of said organic polymers. The foregoing list of pharmaceutically acceptable binding agents is not meant to be exhaustive, but merely illustrative as a person of ordinary skill in the art would understand that many other pharmaceutically acceptable binding agents or combination of binding agents could also be used. Polyethylene glycol 4000 is the preferred pharmaceutically acceptable binding agent.

Examples of suitable pharmaceutically acceptable excipients include gliding agents like magnesium stearate or calcium stearate, stearic acid, talcum and/or starch; fillers like calcium phosphate, corn starch, dextrans, dextrin, hydrated silicon dioxide, microcrystalline cellulose, kaolin, lactose, mannitol, polyvinyl pyrrolidone, precipitated calcium carbonate, sorbitol and/or talcum; disintegrating agents like Aerosil™ (silicic acid), alginic acid, amylose, calcium alginate, calcium carbonate, formaldehyde gelatin, pectic carbonate, sago starch, sodium bicarbonate and/or starch; and/or moisturizers like glycerol and/or starch. The foregoing list of pharmaceutically acceptable excipients is not meant to be exhaustive, but merely illustrative as a person or ordinary skill in the art would understand that many other pharmaceutically acceptable excipients or combination of excipients could also be used. For the purposes of the present disclosure, synthetic oils and monomeric phthalic acid esters are not to be regarded as suitable pharmaceutically acceptable excipients. In one embodiment, the pancreatin micropellets or pancreatrin microspheres contain no pharmaceutically acceptable excipients, but can optionally contain a greater amount of pancreatin.

In one embodiment, the pancreatin micropellets can be prepared by a manufacturing process comprising the steps of:
(a) preparing an extrudable mixture comprising:
   i. 10 % to 95 % pancreatin;
   ii. 5 % to 90 % of at least one pharmaceutically acceptable binding agent;
   iii. 0% to 10% of at least one pharmaceutically acceptable excipient; and
   iv. one or more enzyme-friendly organic solvents in an amount sufficient to form an extrudable mixture;
   wherein the percentages of components are weight to weight of the pancreatin micropellets and the constituents i.), ii.) and iii.) (if present) add to 100 % by weight;
(b) creating pancreatin micropellets from the extrudable mixture;
(c) forming the pancreatin micropellets into approximately spherical or approximately ellipsoidal shape in the presence of additional enzyme-friendly organic solvent; and
(d) removing the one or more enzyme-friendly organic solvents from the pancreatin micropellets such that the pancreatin micropellets are substantially free of the one or more enzyme-friendly organic solvents.

Process variations wherein the pancreatin micropellets are substantially free of synthetic oils are preferred.

Further, process variations wherein the pharmaceutically acceptable excipients are present in an amount of 0% are preferred.

The amounts of pancreatin, pharmaceutically acceptable binding agent(s), pharmaceutically acceptable excipient(s) and/or enzyme-friendly organic solvent may be varied by those skilled in the art to arrive at the pancreatin micropellets having the preferred composition and characteristics as indicated herein.

Enzyme-friendly organic solvents facilitate mixing and other processing procedures and may afterwards be removed, for example, by drying. Typically, after removal of the enzyme-friendly organic solvents, a certain amount of solvent remains in the pancreatin micropellets. The remaining solvent in the micropellets can comprise enzyme-friendly organic solvents, water, or a mixture of enzyme-friendly organic solvents with water. If water is present as a solvent, this will typically have been present in the pancreatin which was used as the starting material. The amount of solvent present in the pancreatin micropellets after removal of the enzyme-friendly organic solvents is typically less than 5 % and normally less than 3 % by weight of the pancreatin micropellet.

Examples of suitable enzyme-friendly organic solvents are acetone, chloroform, dichloromethane or straight-chained or branched C₁₋₄-alcohols, particularly methanol, ethanol, 1-propanol, 2-propanol, 2-butanol, tert-butanol or mixtures of said solvents. 2-propanol is the preferred enzyme-friendly organic solvent. For the purposes of the present disclosure, synthetic oils are not to be regarded as suitable enzyme-friendly organic solvents. The enzyme-friendly organic solvent is typically used in an amount of 15% to 35% by weight, preferably of 20% to 30% by weight, relative to the amount of pancreatin, used. The foregoing list of suitable enzyme-friendly organic solvents is not meant to be exhaustive, but merely illustrative as a person or ordinary skill in the art would understand that many other enzyme-friendly organic solvents or combination of solvents could also be used.The amounts of pancreatin, pharmaceutically acceptable binding agent(s), pharmaceutically acceptable excipient(s) and/or enzyme-friendly organic solvent may be varied by those skilled in the art to arrive at the pancreatin micropellet cores having the preferred composition as indicated herein.

The term "substantially free of enzyme-friendly organic solvents" means that the quantity of enzyme-friendly organic solvents present in the oral dosage form would be less than 5% by weight.

Removal of the one or more enzyme-friendly organic solvents from the oral dosage form means that the oral dosage form is subject to conditions whereby it becomes substantially free from enzyme-friendly organic solvents. Removal of the enzyme-friendly organic solvents can be by any method known to those of ordinary skill in the art. The preferred method is by drying. Drying can e.g. be performed at a temperature from 25°C to 75°C, preferably from 30°C to 55°C. Additionally, removal of the one or more enzyme-friendly organic solvents would also typically result in the oral dosage form containing an amount of water which is less than 5% and typically less than 3% by weight.

In a preferred embodiment of the disclosed process for the manufacture of pancreatin micropellets the pancreatin micropellet cores are created in process step (b) by extrusion. Remarkably, an extrudable mixture is obtained even when the mixture is substantially free of synthetic oils. In process step (b), if the creating of the micropellet cores from the extrudable mixture is accomplished by means of extrusion, then the temperature preferably does not exceed 70°C during extrusion, more preferably the temperature does not exceed 50°C. Also, in the event of extrusion, piercing dies are preferably used which have a hole diameter of 0.5 to 2.0 mm, preferably of 0.7 to 1.5 mm, and more preferably 0.8 mm. Preferably, the pancreatin micropellet or pancreatin microsphere has a diameter of 0.5 to 2.0 mm, in particular of 0.7 to 1.5 mm, e.g. 0.8 mm. If the extrudable mixture is extruded, then the extrudate fragments are brought to a suitable length for the forming step. This can be done e.g. by means of a cutting device arranged downstream to the extruding press in a manner known to the a person of ordinary skill in the art. The forming in process step (c) can be carried out e.g. in a customary rounding apparatus. In the rounding apparatus, the extrudate fragments are then formed into an approximately spherical or approximately ellipsoidal shape in the presence of additional enzyme-friendly organic solvent which may be the same or different than the enzyme-friendly organic solvent used in process step (a).

When prepared substantially free of synthetic oils, processing of the extrudate fragments in the rounding apparatus is improved relative to other known processes which use synthetic oils. For example, a lower amount of enzyme-friendly organic solvent needs to be added when forming the pancreatin micropellets into an approximately spherical or approximately ellipsoidal shape and fewer of the extrudate fragments stick to parts of the rounding apparatus when the process is practiced with an extruder and rounding apparatus.

The invention further provides a process for producing a CRPC which is an enteric coated oral dosage form of an acid-labile drug comprising the steps of:
a. providing an oral dosage form of an acid-labile drug, **wherein the acid-labile drug is pancreatin**;
b. providing an enteric-coating solution comprising
   i. at least one film-forming agent **as defined in claim 9**;
   ii. a plasticizer which is a mixture of cetyl alcohol and triethyl citrate, which are collectively present in an amount of greater than 3 % by weight relative to at least one film-forming agent and wherein the weight to weight ratio of cetyl alcohol to triethyl citrate is from 0.05:1 to 1:1;
   iii. optionally, at least one anti-sticking agent; and
   iv. one or more enzyme-friendly organic solvents;
c. coating the oral dosage form with the enteric-coating solution wherein the product temperature of the oral dosage form during coating is kept at a temperature suitable to apply the enteric-coating solution;
d. drying the coated oral dosage form.

In the foregoing process for producing an enteric-coated oral dosage form of an acid-labile drug, the oral dosage form(s), the film-forming agent(s), the plasticizer(s), the anti-sticking agent(s) and the enzyme-friendly organic solvents generally have the meanings as set forth above.

Process step b.) may be performed at a temperature between 15°C and 60°C. Performing process step b.) at ambient temperature (i.e. room temperature, approximately between 20°C and 30°C), is preferred. The suitable enzyme-friendly organic solvents are acetone, 2-butanol, tert.-butanol, chloroform, dichloromethane, ethanol, methanol, 1-propanol, 2-propanol and mixtures of said solvents. Acetone, ethanol and 2-propanol or their mixtures are preferred as enzyme-friendly organic solvents. Acetone is most preferred.

The enzyme-friendly organic solvent is typically used in an amount between 6 and 10 times, preferably between 7 and 8 times, the weight of the non-solvent coating constituents used to prepare the pancreatin micropellets. For example, if the non-solvent coating constituents make up to a total weight of 1.5 g, then 9 g to 15 g of enzyme-friendly organic solvent may be used in process step a.).

The enteric coating optionally comprises an anti-sticking agent. Suitable anti-sticking agents include dimethicone and castor oil. Dimethicone, in particular dimethicone 1000, is the preferred anti-sticking agent. The anti-sticking agent is usually present in the enteric coating in an amount between 1.5% and 3% by weight relative to the film-forming agent (range limits included). Synthetic oils are not to be regarded as preferred anti-sticking agents. The foregoing list of anti-sticking agents is not meant to be exhaustive but merely illustrative, as a person or ordinary skill in the art would understand that many other anti-sticking agents or combination of anti-sticking agents could also be used.

Due to the process for producing CRPCs, viz. the coating process as described herein, pharmaceutically acceptable residual amounts of the enzyme-friendly organic solvent(s) present in the enteric-coating solution may still be present in the final enteric coated oral dosage form. It is understood that CRPCs comprising pharmaceutically acceptable residual amounts of enzyme-friendly organic solvent(s) are within the scope of the present invention.

In process step c.) the product temperature of the oral dosage form is maintained, while coating, between 32°C and 55°C, more preferred between 35°C and 50°C, most preferably between 37°C and 49°C (all range limits included). In process step c.), when cetyl alcohol or a mixture of cetyl alcohol and triethyl citrate is used, the product temperature of the oral dosage form is preferably maintained between 40°C and 46°C (range limits included). Maintaining the product temperature of the oral dosage form within the preferred temperature ranges while coating results in improved gastric-acid resistant properties of the CRPC, in particular when the enteric coating comprise cetyl alcohol and triethyl citrate as plasticizers. The coating in process step c.) can be accomplished by any process or method known to a person of ordinary skill in the art. Spray coating is preferred. If the coating in process step c.) is performed by spray coating, the spray rate can be between 97 kg/h and 115 kg/h. Usually, process step c.) is performed in a way that the enteric coating comprises between 20% and 30% by weight, preferably between 22% and 26% by weight and more preferably between 22.5 % and 25 % by weight of the total composition of the enteric coated oral dosage form or CRPC. The exact parameters to be applied in process step c.) to achieve the desired enteric coating will depend on the coating technique used. The person skilled in the art understands how to achieve coating films of a desired thickness when using different coating techniques.

Drying of the enteric-coated oral dosage form of the acid-labile drug in process step d.) is usually performed between 30°C and 90°C, preferably between 35°C and 50°C, and for a period of between 1 hour and 60 hours, preferably for a period of between 6 hours and 36 hours.

Disclosed herein is a process for the manufacture of enteric coated pancreatin micropellets, comprising the steps of:
aa. providing uncoated pancreatin micropellets;
bb. providing an enteric-coating solution comprising
   i. at least one film-forming agent **as defined in claim 9**;
   ii. a plasticizer which is a mixture of cetyl alcohol and triethyl citrate, which are collectively present in an amount of greater than 3 % by weight relative to at least one film-forming agent and wherein the weight to weight ratio of cetyl alcohol to triethyl citrate is from 0.05:1 to 1:1;
   iii. optionally, at least one anti-sticking agent; and
   iv. one or more enzyme-friendly organic solvent(s);
cc. coating the uncoated pancreatin micropellets with the enteric-coating solution wherein the product temperature of the pancreatin micropellets during coating is kept at a temperature suitable to apply the enteric-coating solution; and
dd. drying the coated pancreatin micropellets.

In the foregoing process for producing pancreatin micropellets, the film-forming agent(s), the plasticizer(s), the anti-sticking agent(s) and the enzyme-friendly organic solvents generally have the meanings as previously set forth. Preferably, the uncoated pancreatin micropellets which are provided in process step aa.) and which are substantially free of synthetic oils are produced according to the process for the manufacture of pancreatin micropellets as described above.

Due to the process for producing pancreatin micropellets, viz. the coating process as described herein, pharmaceutically acceptable residual amounts of the enzyme-friendly organic solvent(s) present in the enteric-coating solution may still be present in the pancreatin micropellet after drying. It is understood that pancreatin micropellets comprising pharmaceutically acceptable residual amounts of enzyme-friendly organic solvent(s) are within the scope of the present invention.

Process step bb) may be performed at a temperature between 15°C and 60°C. Performing process step bb) at ambient temperature (i.e. room temperature, approximately between 20°C and 30°C), is preferred. The suitable enzyme-friendly organic solvents are acetone, 2-butanol, tert.-butanol, chloroform, dichloromethane, ethanol, methanol, 1-propanol, 2-propanol and mixtures of said solvents. Acetone, ethanol and 2-propanol or their mixtures are preferred as enzyme-friendly organic solvents. Acetone is most preferred.

The enzyme-friendly organic solvent is typically used in an amount between 6 and 10 times, preferably between 7 and 8 times, the weight of the non-solvent coating constituents used to prepare the pancreatin micropellets. For example, if the non-solvent coating constituents make up to a total weight of 1.5 g, then 9 g to 15 g of enzyme-friendly organic solvent may be used in process step bb).

In process step cc.) the product temperature of the pancreatin micropellet is usually maintained, while coating, between 32°C and 55°C, more preferred between 35°C and 50°C, most preferably between 37°C and 49°C. In process step cc), when cetyl alcohol or a mixture of cetyl alcohol and triethyl citrate is used the temperature of the pancreatin micropellet core is maintained between 40°C and 46°C (range limits included). Maintaining the temperature of the pancreatin micropellet cores within the preferred temperature ranges while coating results in improved gastric-acid resistant properties of the pancreatin micropellets, in particular when the enteric coating comprise cetyl alcohol and triethyl citrate as plasticizers. The coating in process step cc.) can be accomplished by any process or method known to a person of ordinary skill in the art. Spray coating is preferred. Usually, process step cc.) is performed in a way that the enteric coating comprises between 20% and 30% by weight, preferably between 22% and 26% by weight and more preferably between 22.5 % and 25 % by weight of the total composition of the pancreatin micropellet. The exact parameters to be applied in process step cc.) to achieve the desired enteric coating will depend on the coating technique used. The person skilled in the art understands how to achieve coating films of a desired thickness when using different coating techniques.

Drying of the enteric-coated pancreatin micropellets in process step dd) is usually performed between 30°C and 75°C, preferably between 30°C and 55°C, more preferably between 35°C and 50°C, and for a period of between 6 hours and 60 hours, preferably for a period of between 10 hours and 36 hours.

The invention further provides a CRPC which is an enteric coated oral dosage form of an acid-labile drug, pancreatin, which is obtainable by the process or its variants described herein. If the CRPC is a pancreatin micropellet or pancreatin microsphere, the preferred diameter is 0.6 to 2.1 mm, more preferred between 0.7 mm and 1.6 mm.

In one embodiment, oral CRPCs are described wherein pancreatin is the acid-labile drug for delivery to an area of the GI tract having a pH greater than the pH of the stomach, specifically to the small intestine, usually to the duodenum, of mammals such as humans. The oral CRPCs comprising pancreatin are particularly suited for the prophylaxis and/or treatment of digestive disorders of different origins like maldigestion and/or for the prophylaxis and/or treatment of pancreatitis, cystic fibrosis, diabetes type I, diabetes type II and/or other conditions resulting from pancreatic exocrine insufficiency in mammals and humans.

Maldigestion in mammals such as humans is usually based on a deficiency of digestive enzymes, in particular on a deficiency of endogenous lipase, but also of protease and/or amylase. The cause of such a deficiency of digestive enzymes is frequently a hypofunction of the pancreas (e.g. pancreatic insufficiency, usually known as pancreatic exocrine insufficiency), the organ which produces the largest quantity of, and the most important, endogenous digestive enzymes. If the pancreatic insufficiency is pathological, it may be congenital or acquired. Acquired chronic pancreatic insufficiency may, for example, result from alcoholism. Congenital pancreatic insufficiency may, for example, result from disease such as cystic fibrosis. The consequences of the deficiency of digestive enzymes may be severe symptoms of under-nutrition and malnutrition, which may be accompanied by increased susceptibility to secondary illnesses. In one specific embodiment, pancreatin micropellets according to the invention are therefore particularly suited for treating pancreatic exocrine insufficiency of any origin.

In another embodiment, an enteric coated oral dosage form of pancreatin is provided as previously described, for the manufacture of a medicament for the treatment of medical conditions such as digestive disorders, pancreatic exocrine insufficiency, pancreatitis, cystic fibrosis, diabetes type I and/or diabetes type II.

In yet another embodiment, a method is provided for the treatment of a medical condition such as digestive disorders, pancreatic exocrine insufficiency, pancreatitis, cystic fibrosis, diabetes type I and/or diabetes type II by administering a therapeutically effective amount of an enteric coated oral dosage form of pancreatin to a person in need of such treatment.

The enteric coating as disclosed herein will usually be applied to oral dosage forms selected from granules, granulates, micropellets, microspheres, microtablets, pellets, pills, powders and/or tablets and said coated oral dosage forms may then be incorporated into uncoated capsules. However, in an alternative embodiment, the invention also comprises enteric coated capsules which contain coated or, more commonly, uncoated oral dosage forms selected from granules, granulates, micropellets, microspheres, microtablets, pellets, pills, powders and/or tablets.

The coated oral dosage forms of the acid-labile drug selected from granules, granulates, micropellets, microspheres, microtablets, pellets, pills, powders and/or tablets or the capsules may further be incorporated into at least one outer package e.g. selected from blisters or bottles. In embodiments of the invention, a pharmaceutical pack or kit is provided comprising one or more containers filled with the ingredients of a pharmaceutical composition of the invention. Associated with such container(s) can be various written materials such as instructions for use, or a notice in the form prescribed by a governmental agency regulating the manufacture, use or sale of pharmaceutical products, which notice reflects approval by the agency of manufacture, use, or sale for human or veterinary administration.

The CRPCs as disclosed herein are substantially free of both monomeric phthalic acid ester plasticizers such as dibutyl phthalate and synthetic oils such as paraffins or mineral oils while providing the desired performance in terms of targeted release and storage stability. Further, the CRPCs presently disclosed, in particular in their preferred embodiments, possess superior gastric acid resisting and protective properties, e.g. superior resisting and protective properties in an acidic environment, specifically at pH 1 and/or pH 5. The enteric coating as proposed for the presently disclosed CPRCs still further provides beneficial properties like dissolution profiles. CRPCs as disclosed herein wherein the plasticizer is comprised of cetyl alcohol and triethyl citrate (CA/TEC-Compositions) are preferred in this regard. Further, CA/TEC-Compositions in general preserve a higher lipase content when pancreatin is the acid-labile drug and usually possess a lower water content compared to CRPCs when other plasticizers are used.

### EXAMPLES

The following examples are meant to be illustrative and not to limit the present disclosure. Other suitable modifications and adaptations are of the variety normally encountered by those skilled in the art and are fully within the spirit and scope of the present disclosure.

### A. Preparation of an enteric coated oral dosage form of an acid-labile drug

### 1. Preparation of uncoated pancreatin micropellets

15.9 kg of pancreatin was mixed with 3.975 kg of polyethylene glycol 4000 in a commercially available high share mixer and thoroughly moistened with 3.975 kg of 2-propanol. The resulting mixture was extruded by means of a commercially available extruding press which was equipped with a piercing die having 0.8 mm internal diameter bores and a cutting device arranged downstream. The temperature was less than 50 °C while pressing. The extruded mass was cut into extrudate fragments of approximately 5 mm length by means of the cutting device.

The resulting 14.64 kg of the extrudate fragments were transferred in four portions of roughly equal size to a commercially available rounding apparatus and rounded off to give approximately elliptically or approximately spherically shaped micropellets. An additional 135 g of 2-propanol was added while rounding.

After drying in a commercially available continuous vacuum dryer (Vötsch type) at a temperature in a range from between 35 °C and 50 °C for 12 hours, the pancreatin micropellets were graded, first with a 3.15 mm sieve (sieving of oversize grain > 3.15 mm) and then with a 0.7 mm sieve (sieving of undersize grain < 0.7 mm) and afterwards with a 1.25 mm sieve (sieving of oversize grain > 1.25 mm) to yield 11.98 kg of (uncoated) pancreatin micropellets having a pancreatin content of 80 % and a bulk density of 0.67 g/ml.

### 2. Enteric coating of pancreatin micropellets

A coating solution was prepared by adding 1623.2 g of hydroxypropyl methylcellulose phthalate (HP 55), 90.2 g of triethyl citrate, 34.3 g of cetyl alcohol and 38.9 g of dimethicone 1000 to 14030 g of acetone at room temperature while stirring.

5025 g of uncoated pancreatin micropellets (prepared analogously to the process as described herein) were fed into a commercially available fluid bed coater and were spray-coated at a spray rate of 97-101 kg/h and an air pressure of 1.7 bar with the coating solution as prepared above until the desired film-thickness of the coating had been reached. The product temperature of the pancreatin micropellets was monitored with a suitable temperature sensor and maintained in the range between 37 °C and 43 °C (range limits included) during coating. The resulting pancreatin micropellets were then dried in a commercially available vacuum dryer (Vötsch type) at a temperature in a range between 35 °C and 50 °C for 12 hours. The dried pancreatin micropellets were then graded, first with a 0.7 mm sieve (sieving of undersize grain < 0.7 mm) and then with a 1.6 mm sieve (sieving of oversize grain > 1.6 mm) to yield 6532 g of enteric coated pancreatin micropellets having a pancreatin content of 60 % The bulk density of the pancreatin micropellets was 0.69 g/ml.

Further pancreatin micropellets were prepared according to the procedure described above and different coatings were applied in a manner similar to the coating process set forth above to yield further CRPCs. The compositions of the further CRPCs and other compositions are set forth in Table 1 along with certain process parameters from their respective coating processes. Composition G can be produced according to processes as described in U.S. Pat. No. 5,378,462. Comparative composition H was prepared according to a process which includes dibutylphthalate used as a plasticizer in the coating. All batches have been produced in laboratory scale unless otherwise noted.

**Table 1: Pancreatin containing compositions**

| | | **Composition** | | | | | |
|---|---|---|---|---|---|---|---|
| **Ingredients mg/capsule** | | **A** | **B** | **C** | **D** | **1** | **2** |
| **Micropellet Cores** | Pancreatin | 150.00 | 150.00 | 150.00 | 150.00 | 150.00 | 150.00 |
| | PEG 4000 | 37.50 | 37.50 | 37.50 | 37.50 | 37.50 | 37.50 |
| **Enteric Coating (film)** | HP 55 | 48.60 | 48.60 | 48.60 | 48.60 | 48.60 | 48.60 |
| | Dimethicone | 1.25 | 1.25 | 1.25 | 1.25 | 1.25 | 1.25 |
| | TEC | 0 | 0 | 3.0 | 4.10 | 5.00 | 0 |
| | CA | 0 | 0.40 | 0 | 0 | 0 | 1.00 |
| | Sum | 237.40 | 237.75 | 240.35 | 241.45 | 242.4 | 238.35 |
| **Process parameters** | Pellet temp. while coating | 40°C | 40°C | 40°C | 40°C | 40°C | 40°C |

| | | **Composition** | | | | | |
|---|---|---|---|---|---|---|---|
| **Ingredients mg/capsule** | | **3** | **4** | **5** | **6*** | **7** | **8** |
| **Micropellet Cores** | Pancreatin | 150.00 | 150.00 | 150.00 | 150.00 | 150.00 | 150.00 |
| | PEG 4000 | 37.50 | 37.50 | 37.50 | 37.50 | 37.50 | 37.50 |
| **Enteric Coating (film)** | HP 55 | 52.60 | 48.60 | 48.60 | 52.25 | 52.25 | 52.25 |
| | Dimethicone | 1.25 | 1.25 | 1.25 | 1.25 | 1.25 | 1.25 |
| | TEC | 0 | 3.60 | 3.00 | 2.90 | 2.90 | 2.90 |
| | CA | 1.15 | 0.40 | 1.00 | 1.10 | 1.10 | 1.10 |
| | Sum | 242.50 | 241.35 | 241.35 | 245.00 | 245.00 | 245.00 |
| **Process parameters** | Pellet temp. while coating | 40°C | 40°C | 40°C | 40 °C | 30 °C | 35 °C |

| | | **Composition** | | | | | |
|---|---|---|---|---|---|---|---|
| **Ingredients mg/capsule** | | **9** | **10** | **11** | **12** | **13** | **14** |
| **Micropellet** | Pancreatin | 150.00 | 150.00 | 150.00 | 150.00 | 150.00 | 150.00 |
| | PEG 4000 | 37.50 | 37.50 | 37.50 | 37.50 | 37.50 | 37.50 |
| **Enteric Coating (film)** | HP 55 | 56.34 | 56.34 | 56.34 | 52.25 | 52.25 | 56.34 |
| | Dimethicone | 1.35 | 1.35 | 1.35 | 1.25 | 1.25 | 1.35 |
| | TEC | 3.13 | 3.13 | 3.13 | 2.90 | 2.90 | 3.13 |
| | CA | 1.19 | 1.19 | 1.19 | 1.10 | 1.10 | 1.19 |
| | Sum | 249.51 | 249.51 | 249.51 | 245.00 | 245.00 | 249.51 |
| **Process parameters** | Pellet temp. while coating | 37 °C | 40 °C | 43 °C | 49 °C | 40 °C | 46 °C |

| **Ingredients mg/capsule** | | **15** | **E** | **F** | **G** | **H** | |
|---|---|---|---|---|---|---|---|
| **Micropellet cores** | Pancreatin | 150.00 | 150.00 | 150.00 | 150.00 | 150.00 | |
| | PEG 4000 | 32.01 | 37.50 | 37.50 | 37.50 | 37.50 | |
| | Light mineral oil | 0 | 0 | 0 | 3.75 | 0 | |
| **Enteric Coating (film)** | HP 55 | 48.10 | 48.60 | 48.60 | 48.60 | 48.60 | |
| | Dimethicone | 1.15 | 1.25 | 1.25 | 1.25 | 1.25 | |
| | TEC | 2.67 | 1.00 | 2.00 | 0 | 0 | |
| | CA | 1.01 | 0 | 0 | 0 | 0 | |
| | DBP | 0 | 0 | 0 | 4.10 | 4.10 | |
| | Light mineral oil | 0 | 0 | 0 | 3.30 | 0 | |
| | Sum | 213.00 | 238.35 | 239.35 | 248.50 | 241.50 | |
| **Process parameters** | Pellet temp. while coating | n.a. | 40°C | 40°C | 40°C | 40°C | |
| Table 1 (continued); | | | | | | | |
| PEG=polyethylene glycol; TEC=triethyl citrate; CA=cetyl alcohol; HP 55=hydroxypropyl methylcellulose phthalate; temp.=temperature; DBP=dibutyl phthalate; *=production scale; n.a: data not available. | | | | | | | |
| **Compositions 4 to 15 only are compositions according to the invention.** | | | | | | | |
| Composition G is a currently available high-quality pharmaceutical composition comprising pancreatin and light mineral oil. | | | | | | | |
| Compositions No. 5, 6, 10, 13, 14 and 15 are preferred examples of compositions containing CA/TEC as the plasticizer. | | | | | | | |
| Composition No. 3 is an example of a composition comprising cetyl alcohol as the sole plasticizer. | | | | | | | |

### B. Determination of the gastric acid resistance of enteric coated pancreatin micropellets at pH 1 and pH 5

Resistance to gastric juice (pH1) of the different pancreatin micropellets from Table 1 was determined by immersing the pancrelipase micropellets for 2 hours in 0.1 mol/l hydrochloric acid in a disintegration tester according to the European Pharmacopoeia (Ph. Eur.). Then the un-dissolved portion of the pellets was separated from the solution and their residual lipase activity was determined according to the lipase assay of Ph. Eur./ The International Pharmaceutical Federation" (FIP), PO Box 84200; 2508 AE The Hague; The Netherlands. The results of these tests for gastric resistance of the enteric coating are presented in Table 2 ("stability at pH1").

Further, a similar test at pH 5 was performed using the same conditions as outlined above, with the exception that a phosphate buffer pH 5.0 (2.0 g sodium chloride and 9.2 g sodium di-hydrogen phosphate monohydrate per liter adjusted to pH 5.0) was used as a solvent instead of 0.1 mol/l hydrochloric acid. The results of these tests for gastric resistance are also presented below in Table 2 ("stability at pH5").

The gastric acid resistances of the compositions from Table 1 (see above) are each given in Table 2 as percentages of the residual lipolytic activity after the incubation in relation to the actual lipolytic activity of the samples tested prior to the incubation (relative gastric acid resistance). The lipolytic activity is determined according to the lipase assay described in the USP monograph "pancrelipase delayed-release capsules". In principle, any standardized and characterized pancreatin sample may be used as the lipase reference standard. For example, a predetermined lipolytic activity standard may be obtained from the "International Pharmaceutical Federation" (FIP), PO Box 84200; 2508 AE The Hague; The Netherlands. For the purposes of the present invention, an internal pancreatin standard was used which is available on request from Solvay Pharmaceuticals GmbH, Hans-Boeckler-Allee 20, 30173 Hannover, Germany.

**Table 2: Relative gastric acid resistances (stabilities) of compositions in Table 1 at pH 1 and pH 5**

| **Composition** | **Stability at pH 5 [%]** | **Stability at pH 1 [%]** |
|---|---|---|
| **A** | 15.3 | 15.9 |
| **B** | 63.2 | 53.8 |
| **C** | 71.6 | 84.2 |
| **D** | 52.0 | 93.6 |
| **1** | 87.0 | 96.0 |
| **2** | 76.4 | 92.6 |
| **3** | 92.1 | 94.5 |
| **4** | 85.3 | 93.7 |
| **5** | 92.0 | 93.0 |
| **6** | 94.9 | 99.4 |
| **7** | 67.4 | 89.8 |
| **8** | 80.5 | 95.2 |
| **9** | 83.8 | 90.8 |
| **10** | 97.9 | 99.6 |
| **11** | 89.0 | 93.5 |
| **12** | 83.7 | 94.8 |
| **13** | 100.2 | 102.7 |
| **14** | 93.6 | 98.7 |
| **E** | 48.6 | 65.0 |
| **F** | 36.5 | 75.0 |
| **G** | 98.6 | 100.6 |

Preferred CRPCs have a gastric acid resistance (stability) at pH 1 of at least 75 %, in particular of at least 85 %, preferably of at least 90 %, more preferred of at least 95 %, relative to a predetermined pancreatin lipolytic activity standard.

Other preferred CRPCs as disclosed herein have a gastric acid resistance at pH 5 of at least 75 %, in particular of at least 85 %, preferably of at least 90 %, more preferred of at least 95 %, relative to a predetermined pancreatin lipolytic activity standard.

CRPCs which are most preferred have a gastric acid resistance at pH 1 of at least 90 % and an additional gastric acid resistance at pH 5 of at least 90 %, relative to a predetermined pancreatin lipolytic activity standard.

### C. Determination of the dissolution profile of enteric coated pancreatin micropellets

The dissolution profile of different compositions from Table 1 (see above) was determined according to a test procedure as described in the United States Pharmacopoeia (USP) monograph "pancrelipase delayed-release capsules" with increased gastric resistance phase which is hereby incorporated by reference.

The determination of the resistance to gastric fluid was performed using gastric juice without enzymes according to USP under standardized conditions (37°C, 100 rpm) for 2 hours in the dissolution apparatus (basket apparatus USP). Then the un-dissolved portion of the enteric coated pancreatin micropellets was separated from the solution and transferred into the paddle apparatus according to USP, filled with phosphate buffer solution at pH 6.0 to determine the dissolution of enzymes. The enteric coated pancreatin micropellets were agitated in a dissolution tester under standardized conditions for usually 90 minutes (see exact timepoints in Table 3 below) at 37 °C and 50 rpm.

The lipase activity was determined after selected time points (see Table 3) according to the lipase assay described in the USP monograph "pancrelipase delayed-release capsules".

Further, a test similar to that described above was performed with a "Mcllvain buffer" (pH 6.0; for preparation mix solution A: 7.098 g Na₂HPO₄ anhydrous and 4 g of bile salts in 1000 ml water with solution B: 5.25 g C₆H₈O₇•H2O and 4 g of bile salts in 100 ml water) instead of a USP-compliant phosphate buffer. All other conditions remained as described above for the USP-compliant phosphate buffer.

The results of the dissolution profile tests are presented below as "% residual lipase activity of actual lipase activity" for the test series performed with USP-compliant phosphate buffer (see Table 3a) and for the test series performed with Mcllvain buffer (see Table 3b).

**Table 3a: Dissolution profiles of the enteric coated pancreatin micropellets in phosphate buffer**

| **Time points [min.]** | **% lipase activity of initial actual activity for each composition No.** | | | | | | |
|---|---|---|---|---|---|---|---|
| | **G** | **2** | **3** | **4** | **5** | **13** | **14** |
| **5** | 0.0 | -- | 3.0 | -- | 0.0 | 4.6 | n.a. |
| **10** | 0.0 | -- | 4.9 | -- | 6.2 | 4.6 | 15.37 |
| **15** | 11.9 | -- | 16.4 | -- | 37.8 | 17.6 | 34.38 |
| **20** | 48.0 | -- | 39.3 | -- | 63.5 | 40.8 | n.a. |
| **25** | 62.3 | -- | 59.0 | -- | 72.4 | 59.8 | n.a. |
| **30** | 73.5 | -- | 67.8 | -- | 80.0 | 66.2 | 73.86 |
| **45** | 77.1 | -- | 80.5 | -- | 84.0 | 76.6 | 84.45 |
| **60** | 79.9 | -- | 77.8 | -- | 84.2 | 81.9 | 81.25 |
| **75** | 78.4 | -- | 77.1 | -- | 78.9 | 79.8 | 80.40 |
| **90** | 78.2 | -- | 72.3 | -- | 77.2 | 77.4 | n.a. |
| n.a: data not available | | | | | | | |

**Table 3b: Dissolution profiles of the enteric coated pancreatin micropellet in Mcllvain buffer**

| **Time points [min.]** | **% lipase activity of initial actual activity for each composition No.** | | | | | |
|---|---|---|---|---|---|---|
| | **G** | **2** | **3** | **4** | **5** | **13** |
| **5** | 0.0 | 1.0 | 0.5 | 0.4 | 0.0 | 0.7 |
| **10** | 0.5 | 8.8 | 1.7 | 7.7 | 4.5 | 1.2 |
| **15** | 6.3 | 39.6 | 9.8 | 39.1 | 30.2 | 8.1 |
| **20** | 23.6 | 60.5 | 24.3 | 62.7 | 65.6 | 24.6 |
| **25** | 47.2 | 68.7 | 40.6 | 79.6 | 79.3 | 43.1 |
| **30** | 66.3 | 75.2 | 58.3 | 84.7 | 85.2 | 58.9 |
| **45** | 88.1 | 76.9 | 75.4 | 86.3 | 87.5 | 83.7 |
| **60** | 91.0 | 74.0 | 80.9 | 84.5 | 85.4 | 87.1 |
| **75** | 88.4 | 73.9 | 81.4 | 80.2 | - | 87.1 |
| **90** | - | 71.2 | 80.6 | - | - | 85.4 |
| **105** | - | - | 77.7 | - | - | - |

For the dissolution profile test results as provided in tables 3a and 3b, a comparison of the compositions no. 2, 3, 4, 5 and 13 was performed in each case with the reference composition "G". Said comparison was based on the "Guidance for Industry", SU-PAC-MR, Modified Release Solid Oral Dosage Forms (September 1997) by calculating the similarity factor (f2). The two acceptance limits for determining similarity of two compared curves were (i) a factor (f2) > 50 and (ii) the average deviation at any dissolution sampling point should not be greater than 15 %.

When applying the above-stated acceptance limits for determining similarity it was found that the dissolution profiles of pancreatin micropellet CRPCs no. 2, 4 and 5 (see Table 1) could not be considered to be similar to the dissolution profile of the reference pancreatin micropellet "G" (see Table 1). However, when applying the above-stated acceptance limits for determining similarity it was found that the dissolution profiles of pancreatin micropellet CRPCs no. 3 and 13 (see Table 1) could be considered to be similar to the dissolution profile of the reference pancreatin micropellet "G" (see Table 1).

### D. Storage stability studies for enteric coated pancreatin micropellet CPRCs

For determining storage stability of different pancreatin micropellets from Table 1 (see above), hard gelatin capsules of size 0 were filled with approx. 497 mg of pancreatin micropellets (see Table 1) and packed into 30 ml HDPE bottles for performing the following test series.

The packed pancreatin micropellets were then stored for 5 months under normal or two different aggravated storage conditions (see below for details) and the residual lipase activity was determined in each case analogously to the instructions of Ph. Eur. The results of these storage stability tests of the CPRCs after 5 months' storage periods are presented below in tables 4a and 4b, respectively ("Lipase").

Resistance to gastric juice (pH1) of the different pancreatin micropellets from Table 1 was also determined after a total storage period of 5 months by immersing pancrelipase delayed-release pellets for 2 hours in 0.1 mol/l hydrochloric acid in a disintegration tester according to the Ph. Eur. (Section 2.9.1. "disintegration"). Then the un-dissolved portion of the pellets was separated from the solution and their residual lipase activity was determined according to the lipase assay of Ph. Eur. (monograph "pancreas powder"). The results of these tests for gastric resistance of the enteric coating after 5 months' storage periods under normal or two different aggravated storage conditions are presented in tables 4a and 4b, respectively ("gastric resistance at pH1").

Further, a similar test at pH 5 was done using the same conditions as outlined in the previous paragraph, with the exception that a phosphate buffer pH 5.0 (2.0 g sodium chloride and 9.2 g sodium di-hydrogen phosphate monohydrate per liter adjusted to pH 5.0) was used as a solvent instead of 0.1 mol/l hydrochloric acid. The results of these tests for gastric resistance of the enteric coating after 5 months' storage periods are presented below in tables 4a and 4b, respectively (gastric resistance at pH5").

**Table 4a: Stability results for select compositions from Table 1 at 30 °C and 65 % rel. humidity (slightly aggravated storage conditions)**

| **Conditions** | **CPRC No.** | **% lipase activity of initial activity** | |
|---|---|---|---|
| | | **Months** | |
| | | 0 | 5 |
| **Lipase (initial activity)** | G | 100 | 92 |
| | 3 | 100 | 88 |
| | 13 | 100 | 94 |
| **Gastric resistance at pH 1 (actual activity)** | G | 101 | 91 |
| | 3 | 95 | 95 |
| | 13 | 103 | 99 |
| **Gastric resistance at pH 5 (actual activity)** | G | 99 | 92 |
| | 3 | 92 | 86 |
| | 13 | 100 | 95 |

**Table 4b: Stability results for select compositions from Table 1 at 40 °C and 75 % rel. humidity (aggravated storage conditions)**

| **Conditions** | **CPRC No.** | **% lipase activity of initial activity** | | | | | |
|---|---|---|---|---|---|---|---|
| | | **Months** | | | | | |
| | | 0 | 1 | 2 | 3 | 4 | 5 |
| **Lipase (initial activity)** | G | 100 | 90 | 80 | 77 | 69 | 64 |
| | 3 | 100 | 87 | 79 | 69 | 64 | 61 |
| | 13 | 100 | 97 | 87 | 81 | 73 | 67 |
| **Gastric resistance at pH 1 (actual activity)** | G | 101 | 96 | 101 | 94 | 96 | 96 |
| | 3 | 95 | 94 | 94 | 96 | 87 | 86 |
| | 13 | 103 | 95 | 97 | 97 | 96 | 89 |
| **Gastric resistance at pH 5 (actual activity)** | G | 99 | 92 | 95 | 76 | 87 | 40 |
| | 3 | 92 | 86 | 78 | 63 | 51 | 22 |
| | 13 | 100 | 90 | 83 | 73 | 43 | 15 |

From the data presented in tables 4a and 4b it can be concluded that the tested composition Nos. G, 3 and 13 (see Table 1) are of satisfactory storage stability under normal and slightly aggravated storage conditions over a 5 months' storage period. The lipase content of composition No. 13, although similar to the two comparative compositions, was best preserved over the observed 5 months' periods under slightly aggravated and aggravated storage conditions.

Under slightly aggravated storage conditions which are most relevant in practice, composition No. 13 performed best in terms of gastric resistance at pH 1 and pH 5 over the observed 5 months' periods.

Where in the present disclosure numeric values are given as ranges, the respective range limits are generally meant to be included in and being part of the given ranges unless expressly stated otherwise.

The use of the terms "a" and "an" and "the" and similar references in the context of this disclosure (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., such as, preferred, preferably) provided herein, is intended merely to further illustrate the content of the disclosure and does not pose a limitation on the scope of the claims. No language in the specification should be construed as indicating any non-claimed element as essential to the practice of the invention.

Accordingly, this invention includes all modifications and equivalents of the subject matter recited in the claims appended hereto as permitted by applicable law. Moreover, any combination of the above described elements in all possible variations thereof is encompassed by the invention unless otherwise indicated herein or otherwise clearly contradicted by context.

## Claims

1. A controlled release pharmaceutical composition comprising an oral dosage form of pancreatin and an enteric coating,
the enteric coating comprising:
a) at least one film-forming agent selected from the group consisting of cellulose acetate phthalate, hydroxypropyl methylcellulose acetate succinate, hydroxypropyl methylcellulose phthalate, methacrylic acid-ethyl methacrylate-copolymer, and mixtures of said film-forming agents;
b) a plasticizer which is a mixture of cetyl alcohol and triethyl citrate, which are collectively present in an amount of greater than 3% by weight relative to the film forming agent and wherein the weight to weight ratio of cetyl alcohol to triethyl citrate is from 0.05:1 to 1:1; and
c) optionally at least one anti-sticking agent.

2. Controlled release pharmaceutical composition according to claim 1, comprising an anti-sticking agent which is present in an amount of 1.5 to 3% by weight relative to the film-forming agent.

3. Controlled release pharmaceutical composition according to claim 2, wherein the anti-sticking agent is dimethicone.

4. Controlled release pharmaceutical composition according to claim 1, wherein the plasticizer is comprised of cetyl alcohol and triethyl citrate which are collectively present in an amount of 4% to 20% by weight in relation to the film-forming agent.

5. Controlled release pharmaceutical composition according to claim 1 wherein the film-forming agent is hydroxypropyl methylcellulose phthalate.

6. Controlled release pharmaceutical composition according to claim 1 wherein the oral dosage form is selected from the group consisting of granules, granulates, microtablets, micropellets, microspheres, pellets, pills, powders and tablets.

7. Controlled release pharmaceutical composition according to claim 1 or claim 6 wherein the oral dosage form is micropellets or microspheres.

8. Controlled release pharmaceutical composition according to claim 1 wherein the enteric coating is between 20% and 30% by weight of the total composition of the controlled release pharmaceutical composition.

9. A process for producing a controlled release pharmaceutical composition, the process comprising the steps of
a. providing an oral dosage form of pancreatin;
b. providing an enteric-coating solution comprising
i. at least one film-forming agent selected from the group consisting of cellulose acetate phthalate, hydroxypropyl methylcellulose acetate succinate, hydroxypropyl methylcellulose phthalate, methacrylic acid-ethyl methacrylate-copolymer, and mixtures of said film-forming agents;
ii. a plasticizer which is a mixture of cetyl alcohol and triethyl citrate which are collectively present in an amount of greater than 3% by weight relative to the film forming agent and wherein the weight to weight ratio of cetyl alcohol to triethyl citrate is from 0.05:1 to 1:1;
iii. optionally, at least one anti-sticking agent; and
iv. one or more enzyme-friendly organic solvent(s) selected from the group consisting of acetone, 2-butanol, tert-butanol, chloroform, dichloromethane, ethanol, methanol, 1-propanol, 2-propanol and mixtures of said solvents;
c. coating the oral dosage form with the enteric-coating solution wherein the product temperature of the oral dosage form during coating is kept at a temperature between 32°C and 55°C; and
d. drying the coated oral dosage form.

10. Process according to claim 9 wherein the film-forming agent is hydroxypropylmethyl cellulose phthalate.

11. Process according to claim 9 wherein the oral dosage form of the acid-labile drug is selected from the group consisting of granules, granulates, microtablets, micropellets, microspheres, pellets, pills, powders and tablets.

12. Process according to claim 9 wherein the oral dosage form of pancreatin is micropellets or microspheres.

13. An enteric coated oral dosage form of pancreatin, obtainable by a process according to claim 9.

14. A use of an enteric coated oral dosage form of pancreatin as defined in claim 13 for the manufacture of a medicament for the treatment of digestive disorders, pancreatic exocrine insufficiency, pancreatitis, cystic fibrosis, diabetes type I and/or diabetes type II.

## Patentansprüche

1. Pharmazeutische Zusammensetzung mit kontrollierter Freisetzung, umfassend eine orale Darreichungsform von Pankreatin und eine magensaftresistente Beschichtung,
wobei die magensaftresistente Beschichtung umfasst:
a) wenigstens einen Filmbildner, der aus der aus Celluloseacetatphthalat, Hydroxypropylmethylcelluloseacetatsuccinat, Hydroxypropylmethylcellulosephthalat, Methacrylsäure-Ethylmethacrylat-Copolymer und Mischungen der Filmbildner bestehenden Gruppe ausgewählt ist,
b) einen Weichmacher, der eine Mischung aus Cetylalkohol und Triethylcitrat ist, die zusammen in einer Menge vorhanden sind, die bezogen auf den Filmbildner mehr als 3 Gewichts-% beträgt, und wobei das Gewicht-zu-Gewicht-Verhältnis von Cetylalkohol zu Triethylcitrat von 0,05:1 bis 1:1 reicht, und
c) wahlweise wenigstens ein Antihaftmittel.

2. Pharmazeutische Zusammensetzung mit kontrollierter Freisetzung nach Anspruch 1, umfassend ein Antihaftmittel, das bezogen auf den Filmbildner in einer Menge von 1,5 bis 3 Gewichts-% vorhanden ist.

3. Pharmazeutische Zusammensetzung mit kontrollierter Freisetzung nach Anspruch 2, wobei das Antihaftmittel Dimethicon ist.

4. Pharmazeutische Zusammensetzung mit kontrollierter Freisetzung nach Anspruch 1, wobei der Weichmacher aus Cetylalkohol und Triethylcitrat besteht, die zusammen bezogen auf den Filmbildner in einer Menge von 4 bis 20 Gewichts-% vorhanden sind.

5. Pharmazeutische Zusammensetzung mit kontrollierter Freisetzung nach Anspruch 1, wobei der Filmbildner Hydroxypropylmethylcellulosephthalat ist.

6. Pharmazeutische Zusammensetzung mit kontrollierter Freisetzung nach Anspruch 1, wobei die orale Darreichungsform aus der aus Körnchen, Granulaten, Mikrotabletten, Mikropellets, Mikrosphären, Pellets, Pillen, Pudern und Tabletten bestehenden Gruppe ausgewählt ist.

7. Pharmazeutische Zusammensetzung mit kontrollierter Freisetzung nach Anspruch 1 oder Anspruch 6, wobei die orale Darreichungsform Mikropellets oder Mikrosphären ist.

8. Pharmazeutische Zusammensetzung mit kontrollierter Freisetzung nach Anspruch 1, wobei die magensaftresistente Beschichtung zwischen 20 und 30 Gewichts-% der Gesamtzusammensetzung der pharmazeutischen Zusammensetzung zur kontrollierten Freisetzung ausmacht.

9. Verfahren zum Herstellen einer pharmazeutischen Zusammensetzung mit kontrollierter Freisetzung, umfassend die Verfahrensschritte:
a. Bereitstellen einer oralen Darreichungsform von Pankreatin,
b. Bereitstellen einer magensaftresistenten Beschichtungslösung, die umfasst:
i. wenigstens einen Filmbildner, der aus der aus Celluloseacetatphthalat, Hydroxypropylmethylcelluloseacetatsuccinat, Hydroxypropylmethylcellulosephthalat, Methacrylsäure-Ethylmethacrylat-Copolymer und Mischungen der Filmbildner bestehenden Gruppe ausgewählt ist,
ii. einen Weichmacher, der eine Mischung aus Cetylalkohol und Triethylcitrat ist, die zusammen in einer Menge vorhanden sind, die bezogen auf den Filmbildner mehr als 3 Gewichts-% beträgt, und wobei das Gewicht-zu-Gewicht-Verhältnis von Cetylalkohol zu Triethylcitrat von 0,05:1 bis 1:1 reicht, und
iii. wahlweise wenigstens ein Antihaftmittel und
iv. ein oder mehrere enzymverträgliche(s) Lösungsmittel, das/die aus der aus Aceton, 2-Butanol, tert-Butanol, Chloroform, Dichlormethan, Ethanol, Methanol, 1-Propanol, 2-Propanol und Mischungen der Lösungsmittel bestehenden Gruppe ausgewählt ist/sind,
c. Beschichten der oralen Darreichungsform mit der magensaftresistenten Beschichtungslösung, wobei die Produkttemperatur der oralen Darreichungsform während des Beschichtens auf einer Temperatur zwischen 32 °C und 55 °C gehalten wird, und
d. Trocknen der beschichteten oralen Darreichungsform.

10. Verfahren nach Anspruch 9, wobei der Filmbildner
Hydroxypropylmethylcellulosephthalat ist.

11. Verfahren nach Anspruch 9, wobei die orale Darreichungsform aus der aus Körnchen, Granulaten, Mikrotabletten, Mikropellets, Mikrosphären, Pellets, Pillen, Pudern und Tabletten bestehenden Gruppe ausgewählt ist.

12. Verfahren nach Anspruch 9, wobei die orale Darreichungsform von Pankreatin Mikropellets oder Mikrosphären ist.

13. Magensaftresistent beschichtete orale Darreichungsform von Pankreatin, die durch ein Verfahren nach Anspruch 9 erhältlich ist.

14. Verwendung einer magensaftresistent beschichteten oralen Darreichungsform von Pankreatin nach Anspruch 13 zur Herstellung eines Medikamentes zur Behandlung von Verdauungsstörungen, exokriner Pankreasinsuffizienz, Pankreatitis, cystischer Fibrose, Diabetes Typ I und/oder Diabetes Typ II.

## Revendications

1. Composition pharmaceutique à libération contrôlée comprenant une forme de dosage orale de pancréatine et un revêtement entérique,
le revêtement entérique comprenant :
a) au moins un agent formant un film choisi dans le groupe constitué de phtalate acétate de cellulose, succinate acétate d'hydroxy-propylméthylcellulose, phtalate d'hydroxypropylméthylcellulose, copolymère d'acide méthacrylique-méthacrylate d'éthyle, et mélanges desdits agents formant un film ;
b) un plastifiant qui est un mélange d'alcool cétylique et de citrate de triéthyle, qui sont collectivement présents dans une quantité supérieure à 3 % en masse par rapport à l'agent formant un film et où le rapport masse à masse d'alcool cétylique à citrate de triéthyle est de 0,05:1 à 1:1 ; et
c) éventuellement au moins un agent anti-adhérence.

2. Composition pharmaceutique à libération contrôlée selon la revendication 1, comprenant un agent anti-adhérence qui est présent dans une quantité de 1,5 à 3 % en masse par rapport à l'agent formant un film.

3. Composition pharmaceutique à libération contrôlée selon la revendication 2, où l'agent anti-adhérence est la diméthicone.

4. Composition pharmaceutique à libération contrôlée selon la revendication 1, où le plastifiant est constitué d'alcool cétylique et de citrate de triéthyle, qui sont collectivement présents dans une quantité de 4 % à 20 % en masse en relation à l'agent formant un film.

5. Composition pharmaceutique à libération contrôlée selon la revendication 1, où l'agent formant un film est le phtalate d'hydroxypropylméthylcellulose.

6. Composition pharmaceutique à libération contrôlée selon la revendication 1, où la forme de dosage orale est choisie dans le groupe constitué de granules, granulés, microcomprimés, micropilules, microsphères, billes, pilules, poudres et comprimés.

7. Composition pharmaceutique à libération contrôlée selon la revendication 1 ou la revendication 6, où la forme de dosage orale est des microbilles ou des microsphères.

8. Composition pharmaceutique à libération contrôlée selon la revendication 1, où le revêtement entérique est de 20 % à 30 % en masse de la composition totale de la composition pharmaceutique à libération contrôlée.

9. Procédé de production d'une composition pharmaceutique à libération contrôlée, le procédé comprenant les étapes de :
a. fourniture d'une forme de dosage orale de pancréatine ;
b. fourniture d'une solution de revêtement entérique comprenant
i. au moins un agent formant un film choisi dans le groupe constitué de phtalate acétate de cellulose, succinate acétate d'hydroxypropylméthylcellulose, phtalate d'hydroxypropylméthylcellulose, copolymère d'acide méthacrylique-méthacrylate d'éthyle, et mélanges desdits agents formant un film ;
ii. un plastifiant qui est un mélange d'alcool cétylique et de citrate de triéthyle qui sont collectivement présents dans une quantité supérieure à 3 % en masse par rapport à l'agent formant un film et où le rapport masse à masse d'alcool cétylique à citrate de triéthyle est de 0,5:1 à 1:1 ;
iii. éventuellement, au moins un agent anti-adhérence ; et
iv. un ou plusieurs solvant(s) organique(s) favorable(s) aux enzymes choisis dans le groupe constitué d'acétone, 2-butanol, tert-butanol, chloroforme, dichlorométhane, éthanol, méthanol, 1-propanol, 2-propanol et mélanges desdits solvants ;
c. revêtement de la forme de dosage orale avec la solution de revêtement entérique où la température de produit de la forme de dosage orale pendant le revêtement est maintenue à une température de 32°C à 55°C ; et
d. séchage de la forme de dosage orale revêtue.

10. Procédé selon la revendication 9, où l'agent formant un film est le phtalate d'hydroxypropylméthylcellulose.

11. Procédé selon la revendication 9, où la forme de dosage orale du médicament acido-labile est choisie dans le groupe constitué de granules, granulés, microcomprimés, microbilles, microsphères, billes, pilules, poudres et comprimés.

12. Procédé selon la revendication 9, où la forme de dosage orale de pancréatine est des microbilles ou des microsphères.

13. Forme de dosage orale revêtue entérique de pancréatine, pouvant être obtenue par un procédé selon la revendication 9.

14. Utilisation d'une forme de dosage orale revêtue entérique de pancréatine selon la revendication 13 pour la fabrication d'un médicament destiné au traitement de troubles digestifs, d'insuffisance d'exocrine pancréatique, de pancréatite, de fibrose cystique, de diabètes de type I et/ou de diabètes de type II.
